(19) Europäisches Patentamt
European Patent Office
Office européen des brevets

(11) Publication number: **0 031 675**
**B1**

(12) **EUROPEAN PATENT SPECIFICATION**

(45) Date of publication of patent specification: **09.02.83**

(51) Int. Cl.³: **C 07 C 2/00, B 01 J 29/28**

(21) Application number: **80304559.0**

(22) Date of filing: **17.12.80**

# (54) Conversion of olefin containing mixtures to gasoline.

(30) Priority: **31.12.79 US 108617**

(43) Date of publication of application:
**08.07.81 Bulletin 81/27**

(45) Publication of the grant of the patent:
**09.02.83 Bulletin 83/6**

(84) Designated Contracting States:
**DE FR GB IT NL**

(56) References cited:
**EP - A - 0 014 059**
**GB - A - 1 446 522**
**US - A - 3 960 978**
**US - A - 4 108 881**
**US - A - 4 112 056**

(73) Proprietor: **MOBIL OIL CORPORATION**
**150 East 42nd Street**
**New York New York 10017 (US)**

(72) Inventor: **Dwyer, Francis Gerard**
**1128 Talleyrand Road**
**West Chester Pennsylvania 19380 (US)**
Inventor: **Garwood, William Everett**
**125 Warwick Road**
**Haddonfield New Jersey 08033 (US)**

(74) Representative: **Cooper, John Anthony et al,**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 031 675 B1

# Conversion of olefin containing mixtures to gasoline.

This invention relates to an improved method of catalytically converting olefins into gasoline fractions. More particularly, this invention involves the conversion of olefinic mixtures into gasoline range product.

It has long been known to contact various hydrocarbon fractions with acidic catalysts generally and, in particular, with solid siliceous acidic catalysts-including those referred to as crystalline aluminosilicate zeolites. Contact of said hydrocarbon feed with said acid catalysts was carried out for a wide variety of reactions including cracking, isomerization, hydrocracking, etc. Representative U.S. specifications disclosing and claiming contacting of various hydrocarbon fractions with crystalline aluminosilicates are Nos. 3,140,249, 3,140,251, 3,140,253, and 3,140,322.

The contact of paraffinic feedstocks with crystalline aluminosilicate zeolites is also known in the art and by far the primary reason for contacting paraffinic materials with zeolites has been for the purpose of cracking them, i.e. converting then to lower molecular weight products. Typical applications in this general field would be the use of crystalline aluminosilicate zeolites for carrying out dewaxing reactions, i.e. the cracking of paraffins to low molecular weight materials. U.S. Patent No. 3,400,072 discloses a dewaxing process with crystalline aluminosilicates generally and U.S. Patent No. 3,700,585, discloses and claims dewaxing operations carried out with crystalline aluminosilicates of the ZSM—5 type.

U.S. Patent 3,960,978 describes a process for making olefinic gasoline blending stock from $C_2$—$C_5$ olefin mixtures utilizing ZSM—5 type crystalline aluminosilicates.

Recently, because of the greater awareness of the problem of environmental control, as well as air pollution, greater impetus has been given to investigations directed towards increasing the octane number of gasoline without the use of lead.

In order to reduce automobile exhaust emissions to meet federal and state pollution requirements, many automobile manufacturers have now equipped the exhaust systems of their vehicles with catalytic converters. Said converters contain catalysts which are poisoned by lead. Since lead has been widely used in the past to boost the octane number of gasoline, refiners now have to turn to alternate means to improve gasoline octane number.

The patent invention is concerned primarily with the preparation of higher molecular weight olefins from the stated feed and represents a significant improvement over prior art olefin conversion processes to gasoline range products in that octane number can be substantially increased.

In accordance with the invention, there is provided an improved process for producing a gasoline fraction containing predominantly olefinic compounds and preferably having a research octane number of 90 or above and having therein no more than about 20% by weight of aromatics, said process comprising contacting a $C_2$—$C_5$ olefin-containing mixture with a catalyst comprising a highly siliceous zeolite having a constraint index, as hereinafter defined, in the approximate range of 1 to 12 and a silica to alumina mole ratio of at least 200. The significance and manner of determination of "constraint index" are described in our C. B. Specification 1,446,522.

The improvement involved in the present invention is to employ such highly siliceous zeolites in lieu of conventional zeolites, e.g. silica to alumina mole ratio of about 70 to 1. In comparison to the use of conventional ZSM—5 zeolites, the highly siliceous zeolites of the instant invention provide a substantial increase in octane number of the liquid product. The process of the instant invention can yield a gasoline fraction with a research octane number with no added lead (R + O) of at least 92.

The present invention is concerned with contacting a feed stream consisting essentially of $C_2$—$C_5$ olefins, with a catalyst comprising highly siliceous zeolites to obtain predominantly higher molecular weight olefins which have good octane numbers and which are excellent gasoline blending stocks. Said highly siliceous zeolites are characterized by a constraint index in the approximate range of 1 to 12 and a silica to alumina mole ratio of at least 200. Alternatively the product from the instant invention can be used as source of olefins for other chemical purposes such as conversion to alcohols, and the like.

The feedstock useful in the instant invention may be, in addition to a pure $C_2$—$C_5$ olefin mixture, mixtures with other hydrocarbons, water, inerts, e.g. nitrogen, and any of a number of feeds from other sources. These other sources include total gas streams from, for example, an FCC, TCC or Riser Cracking unit, a $C_3$ dry gas fraction, a $C_4$ mixture from an unsaturated gas plant, a gas stream from a coking unit and a gas stream from a pyrolysis unit, just to mention a few.

The silica to alumina mole ratio referred to may be determined by conventional analysis. This ratio is meant to represent, as closely as possible, the ratio in the rigid anionic framework of the crystal and to exclude aluminium in the binder or in cationic or other form within the channels. In highly siliceous zeolites, the upper limit of silica to alumina mole ratio is unbounded. ZSM—5, is one such example wherein the silica to alumina mole ratio is at least 5, but can be 100, 1,000, 10,000, or even greater, i.e. up to and including infinity. The highly silliceous zeolites of this invention are characterized by a silica to alumina mole ratio of at least 200 and it is preferred that the silica to alumina mole ratio be higher, e.g. silica to alumina mole ratios of 500 to 1, 1,000 to 1, 1,400 to 1, 1,600 to 1 and greater.

The preferred zeolites, after activation, acquire an intracrystalline sorption capacity for normal

hexane which is greater than that for water, i.e. they exhibit "hydrophobic" properties. It is believed that this hydrophobic character is advantageous in may instances.

Constraint Index (CI) values for some typical substances are:

| | Constraint Index |
|---|---|
| ZSM—4 | 0.5 |
| ZSM—5 | 8.3 |
| ZSM—11 | 8.7 |
| ZSM—12 | 2 |
| ZSM—23 | 9.1 |
| ZSM—35 | 4.5 |
| ZSM—38 | 2 |
| ZSM—48 | 3.4 |
| TMA Offretite | 3.7 |
| Beta | 0.6 |
| H-Zeolen (mordenite) | 0.4 |
| REY | 0.4 |
| Amorphous Silica-Alumina | 0.6 |
| Erionite | 38 |
| Clinoptilolite | 3.4 |

There may be situations where the activity is so low, i.e., silica to alumina mole ratio approaching infinity, that the Constraint Index cannot be adequately measured, if at all. In such situations, Constraint Index is to mean the Constraint Index of the same zeolite (same crystal structure as determined by X-ray diffraction pattern) in an alumina-containing form.

The class of highly siliceous zeolites defined herein is exemplified by ZSM—5, ZSM—11, ZSM—12, ZSM—48 and other similar materials.

Highly siliceous ZSM—5 is described in greater detail in U.S. Patent Re. No. 29,948. The significant (strong) lines of the X-ray diffraction patent of ZSM—5 are as follows:

$$11.1 \pm 0.2$$
$$10.0 \pm 0.2$$
$$3.85 \pm 0.07$$
$$3.71 \pm 0.05$$

Highly siliceous ZSM—11 is more particularly described in U.S. Patent Applications Serial Nos. 003,143 and 003,145.

In U.S. Application Serial No. 003,143, filed January 15, 1979, a highly siliceous ZSM—11 composition can be identified in terms of mole ratios of oxides as follows:

$$(0\text{—}10)M_{2/n}O : (0\text{—}0.5)Al_2O_3 : (100)\ SiO_2$$

wherein M is at least one cation having a valence n, and is further characterized by the X-ray diffraction pattern of ZSM—11, as shown in Table 1 herein.

In the as-synthesized form, this highly siliceous ZSM—11 has a formula, on a water-free basis, in terms of moles of oxides, per 100 moles of silica, as follows:

$$(0\text{—}10)R_2O : (0\text{—}10)M_{2/n}O : (0\text{—}0.5)Al_2O_3 : (100)SiO_2$$

wherein M is an alkali or alkaline earth metal, $R_2O$ is an organic compound of Group VB element of the

Periodic Chart of the Elements, Fisher Scientific Co., Cat. No. 5—702—10, 1978, preferably nitrogen or phosphorous, containing at least one alkyl or aryl group having between 1 and 7 carbon atoms, preferably between 2 and 5, carbon atoms, preferably containing at least one ethyl or butyl group and still more preferably $R_2O$ is a quaternary ammonium compound.

This highly siliceous ZSM—11 can be prepared from a reaction mixture containing a source of silica, $R_2O$, an alkali metal oxide, e.g., sodium, water, and no added alumina, and has a composition, in terms of mole ratios of oxides, falling within the following ranges:

| REACTANTS | | BROAD | PREFERRED |
|---|---|---|---|
| $SiO_2/R_2O$ | = | 5 to 30 | 10 to 20 |
| $M_2O/R_2O$ | = | 0.0 to 6 | 0.09 to 3.0 |
| $H_2O/R_2O$ | = | 100 to 500 | 300 to 400 |

wherein $R_2O$ is the oxide form of an organic compound of an element of Group VB of the Periodic Chart and can be a compund containing one butyl group, M is an alkali or alkaline earth, and maintaining the mixture, at crystallization temperatures, until crystals of the ZSM—11 are formed. As mentioned above, no alumina is added. The only aluminium present occurs as an impurity.

In U.S. Application Ser. No. 003,145, filed January 15, 1979, a highly siliceous ZSM—11 composition can be identified in terms of mole ratios of anhydrous oxides per 100 moles of silica as follows:

$$(0\text{—}10)M_{2/n}O : [(a)Cr_2O_3 + (b)Fe_2O_3 + (c)Al_2O_3] : 100\ SiO_2,$$

wherein M is at least one cation having in valence n, a=0—4, b=0—5, c=0.001—0.5 and is further characterized by the X-ray diffraction pattern of ZSM—11, as shown in Table 1 herein. However, "a" and "b" cannot both be equal to 0 at the same time; when one equals 0 the other must be greater than the value of "c". The chromium and iron oxide need not all occur as $Cr_2O_3$ or $Fe_2O_3$ but are so calculated in the formula.

In the as-synthesized form, this highly siliceous ZSM—11 has a formula, on a water-free basis, in terms of moles of oxides, per 100 moles of silica, as follows:

$$(0\text{—}3)R_2O : (0\text{—}8)M_{2/n}O : [(a)Cr_2O_3 + (b)Fe_2O_3 + (c)Al_2O_3] : 100\ SiO_2,$$

wherein M is an alkali or alkaline earth metal, $R_2O$ is an organic compound of Group VB element of the Periodic Chart, preferably nitrogen or phosphorous, containing at least one alkyl or aryl group having between 1 and 7 carbon atoms, (preferably between 2 and 5 carbon atoms) preferably containing at least one butyl group and still more preferably $R_2O$ is a quaternary ammonium compound containing at least one butyl group, "a" = 0—4, "b" = 0—5, and "c" = 0.001—0.4. However, "a" and "b" cannot both be equal to 0 at the same time. When one equals 0 the other must be greater than 0 and greater than the value of "c".

This highly siliceous ZSM—11 can be prepared from a reaction mixture containing a source of silica, $R_2O$, an alkali metal oxide, e.g. sodium, a chromium or iron compound, water, and no added alumina, and having a composition, in terms of mole ratios of oxides, falling with the following ratios:

| REACTANTS | | BROAD | PREFERRED |
|---|---|---|---|
| $SiO_2/R_2O$ | = | 5 to 30 | 10 to 20 |
| $M_2O/R_2O$ | = | 0 to 6 | 0.9 to 3.0 |
| $(Cr_2O_3 + Fe_2O_3)/R_2O$ | = | 0.2 to 1.0 | 0.2 to 0.4 |
| $H_2O/R_2O$ | = | 100 to 500 | 300 to 400 |

wherein $R_2O$ is the oxide form of an organic compound of an element of Group VB of the Periodic Chart and can be a compound containing one butyl group, and M is an alkali or alkaline earth metal, and maintaining the mixture until crystals of the ZSM—11 are formed. As mentioned above, no alumina is added. the only aluminum present occurs as an impurity.

TABLE 1
CHARACTERISTIC LINES OF ZSM—11

| Interplanar Spacing D (a) | Relative Intensity |
|---|---|
| 11.2 ± .2 | m. |
| 10.1 ± .2 | m. |
| 6.73 ± .2 | w. |
| 5.75 ± .1 | w. |
| 5.61 ± .1 | w. |
| 5.03 ± .1 | w. |
| 4.62 ± .1 | w. |
| 4.39 ± .08 | w. |
| 3.86 ± .07 | vs. |
| 3.73 ± .07 | m. |
| 3.49 ± .07 | w. |
| (3.07, 3.00) ± .05 | w. |
| 2.01 ± .02 | w. |

The parenthesis around lines 3.07 and 3.00 indicate that they are separate and distinct lines, but are often superimposed. These values were determined by standard technique. The radiation was the K-alpha doublet of copper, and a diffractometer equipped with a scintillation counter (or a geiger counter spectrometer) and a strip chart pen recorder can be used. The peak heights, I, and the positions as a function of 2 theta, where theta is the Bragg angle, were read from the diffractometer chart. From these, the relative intensities, $100I/I_o$, where $I_o$ is the intensity of the strongest line or peak, and d (obs.), the interplanar spacing in Å, corresponding to the recorded lines, were calculated. The intensity in Table 1 is expressed as follows:

m = medium, w = weak and vs = very strong.

ZSM—11 is similar to ZSM—5 with the notable exception that whereas ZSM—5 contains a doublet at about 10.1, 3.73, 3.00 and 2.01 Å interplanar spacing, ZSM—11 shows a singlet at these values. This means that the crystal class of ZSM—11 is different from that of the other zeolites. ZSM—11 is tetragonal whereas ZSM—5 tends to be orthorhombic.

The sodium form as well as other cationic forms reveal substantially the same pattern with minor shifts in interplanar spacing and variation of relative intensity.

Other minor variations can occur depending on the silicon to aluminum mole ratio of the particular sample as well as on its degree of thermal treatment.

Highly siliceous ZSM—12 is more particularly described in U.S. Patent Applications, Serial Nos. 003,146 and 003,144.

In U.S. Application Ser. No. 003,146, filed January 15, 1979, a highly siliceous ZSM—12 composition in its calcined form can be identified, in terms of moles of oxides per 100 moles of silica as follows:

$$(0\text{—}10)M_{2/n}O : (0\text{—}0.5)Al_2O_3 : 100\ SiO_2$$

wherein M is at least one cation having a valence n, and is further characterized by the X-ray diffraction pattern of ZSM—12, as shown in Table 2 herein.

In the as-synthesized form, this highly siliceous ZSM—12 has the formula, on a water-free basis, in terms of moles of oxides per 100 moles of silica, as follows:

$$(0\text{—}10)R_2O : (0\text{—}10)M_{2/n}O : (0\text{—}0.5)Al_2O_3 : (100)\ SiO_2$$

wherein $R_2O$ is the tetraethyl derivative of an element of Group VB, e.g. N, P, As, Sb, preferably N or P, more preferably N, and M is an alkali or alkaline earth metal.

This highly siliceous ZSM—12 can be prepared from a reaction mixture containing a source of silica, $R_2O$, an alkali metal oxide, e.g. sodium, water and no added alumina, and having a composition in terms of mole ratios of oxides, falling within the following ratios:

| REACTANT | | BROAD | PREFERRED |
|---|---|---|---|
| $SiO_2/R_2O$ | = | 2 to 50 | 4 to 23 |
| $M_2O/R_2O$ | = | 0.0 to 8.0 | 0.1 to 2.4 |
| $H_2O/R_2O$ | = | 80 to 500 | 100 to 400 |

wherein $R_2O$ is the oxide form of the tetraethyl derivative of an element of Group VB of the Periodic Chart and M is alkali or alkaline earth metal and maintaining the mixture at crystallization temperature until crystals of the ZSM—12 are formed. As mentioned above, no alumina is added. The only aluminum present occurs as an impurity.

In U.S. Application Ser. No. 003,144, filed January 15, 1979, a highly siliceous ZSM—12 composition in its anhydrous form can be identified, in terms of moles of oxides per 100 moles of silica as follows:

$$(0—8)M_{2/n}O; [(a)Cr_2O_3 + (b)Fe_2O_3 + (c)Al_2O_3] : 100\ SiO_2,$$

in the dehydrated state, wherein M is at least one cation having a valence n, "a" = 0—4, "b" = 0—5, "c" = 0.001—0.5 and is further characterized by the X-ray diffraction pattern of ZSM—12, as shown in Table 2 herein. The chromium and iron need not all occur as $Cr_2O$, or $Fe_2O_3$ but are so calculated in the formula. However, "a" and "b" cannot both be equal to O at the same time. When one is zero, the other must be greater than the value of "c".

This highly siliceous ZSM—12 can be prepared from a reaction mixture containing a source of silica, $R_2O$, and alkali metal oxide, e.g. sodium, a chromium or iron compound, water, and no added alumina, and having a composition, in terms of mole ratios of oxides, falling within the following ratios:

| REACTANTS | | BROAD | PREFERRED |
|---|---|---|---|
| $SiO_2/R_2O$ | = | 2 to 50 | 4 to 23 |
| $M_2O/R_2O$ | = | 0.0 to 8.0 | 0.1 to 2.4 |
| $(Cr_2O_3 + Fe_2O_3)/R_2O$ | = | 0.01 to 1.0 | 0.01 to 0.3 |
| $H_2O/R_2O$ | = | 80 to 500 | 100 to 400 |

wherein $R_2O$ is the oxide form of an organic compound of an element of Group VB of the Periodic Chart and can be an organic compound containing at least one ethyl group and M is alkali or alkaline earth metal and maintaining the mixture at crystallization temperature until crystals of the ZSM—12 are formed. As mentioned above, no alumina is added. The only aluminum present occurs as an impurity.

TABLE 2
CHARACTERISTIC LINES OF ZSM—12

| Interplanar Spacing D (A) | Relative Intensity |
|---|---|
| 11.9 ± 0.2 | m. |
| 10.1 ± 0.2 | m. |
| 4.76 ± 0.1 | w. |
| 4.29 ± 0.08 | vs. |
| 3.98 ± 0.08 | m. |
| 3.87 ± 0.07 | vs. |
| 3.49 ± 0.07 | w. |
| 3.38 ± 0.07 | m. |
| 3.20 ± 0.06 | w. |
| 3.05 ± 0.05 | w. |
| 2.54 ± 0.03 | w. |

These values were determined by standard techniques. The radiation was the K-alpha doublet of copper and a diffractometer equipped with a scintillation counter and a strip chart pen recorder was used. The peak heights, I, and the positions as a function of 2 theta, where theta is the Bragg angle, were read from the diffractometer chart. From these, the relative intensities, 100 $I/I_o$, where $I_o$ is the intensity of the strongest line or peak, and d(obs.), the interplanar spacing in A, corresponding to the recorded lines, were estimated. In Table 2, the relative intensities are given in terms of the symbols m = medium, w = weak and vs = very strong. It should be understood that this X-ray diffraction pattern is characteristic of all the species of ZSM—12 compositions. The sodium form as well as other cationic forms reveal substantially the same pattern with some minor shifts in interplanar spacing and variation in relative intensity. Other minor variations can occur depending on the silicon to aluminum mole ratio of the particular sample, as well as its degree of thermal treatment.

Crystallization of the aforementioned substances described in U.S. Application Ser. Nos. 003,143; 003,145; 003,146; and 003,144 can be generally carried out at either static or stirred conditions. Static conditions can be achieved using polypropylene jars at about 100°C or teflon-lined stainless steel autoclaves at about 160°C. Static conditions can also be carried out under pressure in a static bomb reactor. The total useful range of temperatures is about 80°C to about 180°C for about 6 hours to 150 days. Thereafter, the zeolites are separated from the liquid and recovered. The composition can be prepared utilizing materials which supply the appropriate oxides. Depending on the particular zeolite formulation desired, reaction mixtures can include sodium, silicate, silica hydrosol, silica gel, silicic acid, sodium hydroxide, chromic potassium sulfate, or ferric ammonium sulfate. The organic compounds can include any element of Group VB such as nitrogen, phosphorus, arsenic, antimony, or bismuth, preferably nitrogen or phosphorous. However, in the case of Application Ser. No. 003,146, the organic compounds include the tetraethyl derivatives of Group VB elements.

The preferred compounds are quaternary compounds generally expressed by the following formula:

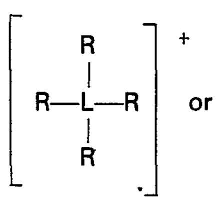 or $R_4L^+$

wherein "L" is an element of Group-B of the Periodic Chart, preferably nitrogen, and each "R" is an alkyl or aryl group having between 1 and 7 (preferably between 2 and 5) carbon atoms. It may be preferable in some formulations that at least one "R" group be an ethyl group or a butyl group.

Normally each alkyl or aryl group will be the same, however, it is not necessary that each group have the same number of carbon atoms in the chain. The oxide of the quaternary compound is

generally supplied by introducing into the reaction mixture a composition such as the tetraethyl (or tetrabutyl as the case may be) hydroxide or chloride of the desired VB element. In preparing an ammonium species, the organic substituted chloride, bromide, or hydroxide is useful. In preparing the phosphonium species of the zeolite, tetraethyl (or tetrabutyl as the case may be) phosphonium chloride is particularly desirable as a means of incorporating the quaternary compound in the zeolite. The other elements of Group VB behave similarly and thus zeolites containing the same can be prepared by the same manipulative procedure substituting another Group VB metal for nitrogen. It should be realized that the oxide can be supplied from more than one source. The reaction mixture can be prepared either batchwise or continuously. Crystal size and crystallization time of the zeolite composition will vary with the nature of the reaction mixture employed and the crystallization conditions.

The quaternary compounds need not be used as such. They may be produced *in situ* by the addition of the appropriate precursors. These precursors comprise a compound characterized by the formula $R_1R_2R_3L$ where $R_1$, $R_2$ and $R_3$ are selected from alkyl, substituted alkyl, aryl, substituted aryl, cycloalkyl, substituted cycloalkyl and hydrogen and L is an element of Group VB and a compound of the formula $R_4L$ where $R_4$ is alkyl, substituted alkyl, cycloalkyl, substituted cycloalkyl, aryl and substituted aryl and L is an electronegative group. According to a special embodiment, the method of the zeolite preparation can be practiced using the compound $R_1R_2R_3L$ alone. Thus, in specific embodiments one may use as the source of $R_2O$, amines or phosphines either primary, secondary or tertiary as well as diamines without addition of any $R_4X$.

Zeolite preparation is facilitated by the presence of at least 0.001%, preferably at least 0.01%, and still more preferably at least 0.1% seed crystals (based on total weight of crystalline product).

ZSM—48 is described in U.S. Application Ser. No. 003,142, filed January 18, 1979.

ZSM—48 can be identified, in terms of moles of anhydrous oxides per 100 moles of silica as follows:

$$(0 \text{ to } 1\text{—}15)RN : (0 \text{ to } 1.5)M_{2/n}O : (0 \text{ to } 2)Al_2O_3 : (100)\ SiO_2$$

wherein M is at least one cation having a valence n, RN is a $C_1$—$C_{20}$ organic compound having at least one amine functional group of $pK_a \geq 7$, and wherein the composition is characterized by the distinctive X-ray diffraction pattern as shown in Table 3 below.

It is recognized that, particularly when the composition contains tetrahedral, framework aluminum, a fraction of the amine functional groups may be protonated. The doubly protonated form, in conventional notation, would be $(RNH)_2O$ and is equivalent in stoichiometry to $2RN + H_2O$.

The X-ray diffraction pattern of ZSM—48 has the following significant lines:

TABLE 3

CHARACTERISTIC LIINES OF ZSM—48

| d | Relative Intensity |
|---|---|
| 11.9 | W—S |
| 10.2 | W |
| 7.2 | W |
| 5.9 | W |
| 4.2 | VS |
| 3.9 | VS |

These values were determined by standard techniques. The radiation was the K-alpha doublet of copper, and a scintillation counter spectrometer with a strip chart pen recorder was used. The peak heights, I, and the positions as a function of 2 times theta, where theta is the Bragg angle, were read from the spectrometer chart. From these, the relative intensities, 100 $I/I_0$, where $I_0$ is the intensity of the strongest line or peak, and d (obs.), the interplaner spacing in A, corresponding to the recorded lines, were calculated. In Table 3 the relative intensities are given in terms of the symbols W = weak, VS = very strong and W—S = weak-to-strong. Ion exchange of the sodium ion with cations reveals substantially the same pattern with some minor shifts in interplanar spacing and variation in relative intensity. Other minor variations can occur depending on the silica to alumina mole ratio of the particular sample, as well as if it has been subjected to thermal treatment.

Highly siliceous ZSM—48 can be prepared from a reaction mixture containing a source of silica,

RN, an alkali metal oxide, e.g. sodium and water, and having a composition, in terms of mole ratios of oxides, falling within the following ranges:

| REACTANTS | BROAD | PREFERRED |
|---|---|---|
| $Al_2O_3/SiO_2$ | =0 to 0.02 | 0 to 0.01 |
| $Na/SiO_2$ | =0 to 2 | 0.1 to 1.0 |
| $RN/SiO_2$ | =0.01 to 2.0 | 0.05 to 1.0 |
| $OH^-/SiO_2$ | =0 to 0.25 | 0 to 0.1 |
| $H_2O/SiO_2$ | =10 to 100 | 20 to 70 |
| $H+(added)/SiO_2$ | =0 to 0.2 | 0 to 0.05 |

wherein RN is a $C_1$—$C_{20}$ organic compound having amine functional group of $pK_a \geq 7$, and maintaining the mixture at 80—250°C until crystals of ZSM—48 are formed. H+(added) is moles acid added in excess of the moles of hydroxide added. In calculating H+(added) and OH values, the term acid (H+) includes both hydronium ion, whether free or coordinated and aluminum. An amine hydrochloride would be a mixture of amine and HCl. In preparing the highly siliceous form of ZSM—48 no alumina is added. The only aluminum present occurs as an impurity.

Preferably, crystallization is carried out under pressure in an autoclave or static bomb reactor, at 80 to 250°C. Thereafter, the crystals are separated from the liquid and recovered. The composition can be prepared utilizing materials which supply the appropriate oxide. Such compositions include sodium silicate, silica hydrosol, silica gel, silicic acid, RN, sodium hydroxide, sodium chloride, etc. RN is a $C_1$—$C_{20}$ organic compound containing at least one amine functional group of $pK_a \geq 7$ and includes such compounds as $C_3$—$C_{18}$ primary, secondary, and tertiary amines, cyclic amines, such as piperidine, pyrrolidone and piperazine, and polyamines such as $NH_2$—$C_nH_{2n}$—$NH_2$ wherein n is 4—12.

Many of the specific zeolites described, when prepared in the presence of organic cations, are unsuitable for use herein, possibly because the intra-crystalline free space is occupied by organic cations from the forming solution. Such zeolites may be made suitable by heating in an inert atmosphere at 540°C for one hour, for example, followed by base exchange with ammonium salts followed by calcination at 540°C in air. The presence of organic cations in the forming solution may not be absolutely essential to the formation of this type zeolite; however, the presence of these cations does appear to favor the formation of many members of this special class of zeolite. More generally, it is desirable to activate this type zeolite by base exchange with ammonium salts followed by calcination in air at about 540°C for from about 15 minutes to about 24 hours.

Natural zeolites may sometimes be converted to this type zeolite by various activation procedures and other treatments such as base exchange, steaming, alumina extraction and calcination, alone or in combinations. Natural minerals which may be so treated include ferrierite, brewsterite, stilbite, dachiardite, epistilbite, heulandite, and clinoptilolite.

In a preferred aspect, the zeolites hereof are selected as those having a crystal framework density, in the dry hyrogen form, of not less than about 1.6 grams per cubic centimeter.

Crystal framework densities of some typical zeolites, including some which are not within the purview of this description, are:

| | Void Volume | Framework Density |
|---|---|---|
| Ferrierite | 0.28 cc/cc | 1.76 g/cc |
| Mordenite | .28 | 1.7 |
| ZSM—5, —11 | .29 | 1.79 |
| ZSM—12 | — | 1.8 |
| ZSM—23 | — | 2.0 |
| Dachiardite | .32 | 1.72 |
| L | .32 | 1.61 |
| Clinoptilolite | .34 | 1.71 |
| Laumontite | .34 | 1.77 |
| ZSM—4 (Omega) | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.45 |
| A | .5 | 1.3 |
| Y | .48 | 1.27 |

One embodiment of this invention resides in the use of a porous matrix together with highly siliceous zeolites of this invention. The highly siliceous zeolites can be combined, dispersed or otherwise intimately admixed with a porous matrix in such proportions that the resulting product contains from 1% to 95% by weight, and preferably from 20 to 80% by weight, of the highly siliceous zeolite in the final composite.

The term "porous matrix" includes inorganic compositions with which the highly siliceous zeolites can be combined, dispersed or otherwise intimately admixed, wherein the matrix may be active or inactive. It is to be understood that the porosity of the compositions employed as a matrix can either be inherent in the particular material or it can be introduced by mechanical or chemical means. Representative matrices which can be employed include metals and alloys thereof, sintered metals and sintered glass, asbestos, silicon carbide aggregates, pumice, firebrick, diatomaceous earths, and inorganic oxides. Inorganic compositions especially those of a siliceous nature are preferred. Of these matrices, inorganic oxides such as clay, chemically treated clay, silica, silica-alumina, etc. are particularly preferred because of their superior porosity, attrition resistance and stability.

The compositing of the highly siliceous zeolites of this invention with an inorganic oxide can be achieved by several methods wherein the highly siliceous zeolites are reduced to a particle size less than 40 microns, preferably less than 10 microns, and intimately admixed with an inorganic oxide while the latter is in a hydrous state such as in the form of hydrosol, hydrogel, wet gelatinous precipitate, or in a dried state, or a mixture thereof. Thus, finely divided highly siliceous zeolites can be mixed directly with a siliceous gel formed by hydrolyzing a basic solution of alkali metal silicate with an acid such as hydrochloric, sulfuric, acetic, etc. The mixing of the three components can be accomplished in any desired manner, such as in a ball mill or other types of mills. The highly siliceous zeolites also may be dispersed in a hydrosol obtained by reacting an alkali metal silicate with an acid or alkaline coagulant. The hydrosol is then permitted to set in mass to a hydrogel which is thereafter dried and broken into

pieces of desired shape or dried by conventional spray drying techniques or dispersed through a nozzle into a bath of oil or other water-immiscible suspending medium to obtain spheroidally shaped "bead" particles of catalyst such as described in U.S. Patent No. 2,384,946. The siliceous gel thus obtained is washed free of soluble salts and thereafter dried and/or calcined as desired.

In a like manner, the highly siliceous zeolites may be incorporated with an aluminiferous oxide. Such gels and hydrous oxides are well known in the art and may be prepared, for example by adding ammonium hydroxide, ammonium carbonate, etc. to a salt of aluminum, such as aluminum chloride, aluminum sulfate, aluminum nitrate, etc. in an amount sufficient to form aluminum hydroxide which, upon drying, is converted to alumina. The highly siliceous zeolites may be incorporated with the aluminiferous oxide while the latter is in the form of hydrosol, hydrogel, or wet gelatinous precipitate or hydrous oxide, or in the dried state.

The inorganic oxide may also consist of raw clay or a clay mineral which was been treated with an acid medium to render it active. The highly siliceous zeolites can be incorporated into the clay simply by blending the two and fashioning the mixture into desired shapes. Suitable clays include attapulgite, kaolin, sepiolite, polygarskite, kaolinite, halloysite, plastic ball clays, bentonite, montmorillonite, illite, chlorite, etc.

Other useful matrices include powders of refractory oxides, such as alumina, alpha alumina, etc., having very low internal pore volume. Preferably, these materials have substantially no inherent catalytic activity of their own.

Conversion conditions for the process of the present invention comprise a temperature from between about 450°F (233°C) and about 800°F (427°C), preferably from between about 500°F (260°C) and about 700°F (372°C); an olefin partial pressure from between about 0.5 psia (4 kPa) and about 100 psia (690 Pa), preferably from between about 1 psia (7 kPa) and about 50 psia (345 kPa), and a WHSV from between about 0.1 and about 25, preferably from between about 0.5 and about 15.

The following examples will serve to illustrate the invention. It is to be understood that these examples are merely illustrative and are not to be construed as limiting the scope of the invention.

## Example 1

Highly siliceous ZSM—5 with a silica to alumina mole ratio of about 1600 to 1 was prepared according to the procedure as set out below.

Prereacted organics preparation

The following materials were charged to an autoclave: 0.30 parts methylethyl ketone, 0,18 parts tri-n-propylamine and 0.15 parts n-propyl bromide. The contents were mixed with gentle agitation for 15 minutes. The agitation was stopped and 1 part water was charged to the autoclave. The autoclave was sealed and heated to 220°F (105°C) and held at 220°F (105°C) for 15 hours. After this reaction period the temperature was raised to 320° (160°C) and the unreacted organics were flashed off. The aqueous phase was removed containing the prereacted organics and contained 1.44% weight nitrogen.

Zeolite Synthesis

Solution Preparation

Silicate Solution

1 part Q-brand sodium silicate
0.58 parts $H_2O$
0.0029 parts Daxad 27

Acid Solution

| | |
|---|---|
| 0.10 parts | $H_2SO_4$ |
| 0.045 parts | NaCl |
| 0.56 parts | prereacted organics |
| 0.16 parts | $H_2O$ |

Additional Solids

0.14 parts NaCl

Additional Liquid

0.029 parts $H_2O$

Procedure

The silicate solution and acid solution were mixed in a mixing nozzle to form a gel which was

discharged into an autoclave to which 0.029 parts water had been previously added. The gel was whipped by agitation and 0.14 parts NaCl were added and thoroughly blended. The autoclave was sealed and heated to ~220°F (105°C) with agitation at 90 rpm and held for 54.3 hours until crystallization was complete. The contents of the autoclave were cooled and discharged. The crystallized product was analyzed by X-ray diffraction and was found to be 100% weight ZSM—5. The chemical analysis of the thoroughly washed crystalline product is:

| | % Wt. | Mole Ratio |
|---|---|---|
| $Al_2O_3$ | 0.10 | 1.0 |
| $SiO_2$ | 98.3 | 1670 |
| Na | 1.6 | — |
| $Na_2O$ | — | 35.5 |
| N | 0.75 | 63.9 |
| C | 8.98 | 892 |

Catalyst Preparation

After drying, the zeolite was mixed with alpha alumina monohydrate and water to an extrudable consistency and formed into 1/16" extrudates. The extrudates were dried, calcined in flowing $N_2$ for 3 hours at 1000°F (538°C) then ion exchanged twice with 1 N $NH_4NO_3$ solution (5 parts $NH_4NO_3$ solution/1 part zeolite) for 1 hour at ambient temperature and dried.

Example 2

Conventional HZSM—5 catalyst with a silica to alumina mole ratio of about 70 to 1 was prepared as follows:

A sodium silicate solution was prepared by mixing 7.6 parts water, 9.1 parts ice and 28.9 parts sodium silicate (28.7 wt.% $SiO_2$, 8.9 wt.% $Na_2O$, 62.4 wt.% $H_2O$) followed by addition of 0.08 parts Daxad 27 (W.R. Grace Chemical Division).

The organic salt solution was prepared by mixing 0.30 parts MEK, 0.18 parts tri-n-propylamine and 0.15 parts n-propyl bromide per part water. The mixture was reacted at about 100—110°C for 17 hours.

An acid solution was prepared by mixing 12.1 parts of the organic solution with 2.4 parts water and 6.0 parts ice followed by 1 part alumina sulfate (17.2 wt.% $Al_2O_3$), 4 parts sulfuric acid (~97 wt.% $H_2SO_4$) and 1.3 parts NaCl.

These solutions were mixed in an agitated vessel and 4.1 parts NaCl were added. The gel molar ratios expressed as oxides are the following:

$SiO_2/Al_2O_3$ — 82
$Na_2O/Al_2O_3$ — 52

The mixture was heated to about 95—110°C. When more than 65% of the gel was crystallized, the temperature was increased to 150—170°C and held there until crystallization was complete. Unreacted organics were removed by flashing and the contents cooled.

The zeolite was washed by decantation and dried. The dried zeolite was then mixed with alumina and water. It was extruded into 1/16" pellets and dried. The extruded material contained 65 parts ZSM—5 per 35 parts alumina.

The dried extrudate was calcined for three hours at 538°C in flowing nitrogen. After cooling, the extrudate was contacted with an ammonium nitrate exchange solution (about 0.4 lb. $NH_4NO_3$/lb. extrudate) for one hour at ambient temperature. This exchange was repeated until the sodium level was less than 0.05 wt.%. After this exchange, the extrudate was washed, dried and calcined in flowing air at 538°C for three hours.

Examples 3 to 5

In Examples 3 to 5, conventional HZSM—5 catalyst in extrudate form with a silica to alumina mole ratio of about 70 to 1 prepared according to Example 2 was employed. The chargestock was a propylene-butylene mixture of the following composition: 57/43 olefin blend of propylene/butylene.

The olefinic feedstock was contacted with 2.65 grams of the conventional HZSM—5 catalyst in a 5/16" stainless steel reactor at atmospheric pressure with a WHSV of about 2.

It was necessary to start at a low temperature (about 450°F [233°C]) with this catalyst to avoid excessive aromatic formation (with resulting low volume yield). At this temperature the liquid product

contained considerable material boiling above the gasoline range (90% B.P. 484°F [252°C]). As the catalyst coked up and the temperature was raised and the gasoline product became acceptable.

Results for Examples 3 to 5 are given in Table 4.

Example 6

In this example, the catalyst prepared according to Example 1 was reacted in accordance with the same conditions as used in Examples 3 to 5 and with the same feedstock as employed in Examples 3 to 5. The results for Example 6 are illustrated in Table 4.

In using high siliceous ZSM—5, it was not necessary to start at a low temperature, as was the case for conventional ZSM—5. At an initial temperature of about 540°F (283°C), olefin conversion was high, aromatic content low (less than 1 weight percent) and gasoline product properties were excellent (liquid product with 90% B.P. at 388°F (198°C) with an octane number of 94 R+O).

By comparison of Example 5 (conventional HZSM—5), with Example 6 (highly siliceous HZSM—5), the improvement attainable by use of the process of the instant invention is readily demonstrated. The octane number obtained using highly siliceous HZSM—5 is three numbers higher than that obtained using conventional HZSM—5. This represents a substantial increase in octane number.

TABLE 4

| | Conventional HZSM—5 Extrudate with $SiO_2/Al_2O_3$ of 70/l | | | Highly siliceous HZSM—5 Extrudate with $SiO_2/Al_2O_3$ of 1600/l |
|---|---|---|---|---|
| Example No. | 3 | 4 | 5 | 6 |
| Temp, °F(°C), Average | 443(229) | 470(244) | 497(259) | 537(281) |
| Max. | 453(239) | 491(255) | 528(276) | 544(285) |
| WHSV | 2.3 | 2.2 | 2.2 | 1.8 |
| Run Time Hours | 4 | 18 | 23.5 | 23 |
| Conversion, wt.% | | | | |
| Propylene | 45 | 58 | 79 | 94 |
| Butylene | 24 | 38 | 50 | 79 |
| Yields, wt.% | | | | |
| $C_1+C_2$ | <0.1 | <0.1 | <0.1 | <0.1 |
| $C_3$'s, Total | 32.2 | 24.4 | 12.5 | 5.4 |
| Propylene | 31.8 | 24.1 | 12.1 | 4.7 |
| Propane | 0.4 | 0.3 | 0.4 | 0.7 |
| $C_4$'s, Total | 32.1 | 26.7 | 21.6 | 13.3 |
| iso-butane | 0.4 | 0.3 | 0.4 | 0.7 |
| Butylene | 31.7 | 26.4 | 21.1 | 12.2 |
| n-Butylene | <0.1 | <0.1 | 0.1 | 0.4 |
| $C_5$'s, Total | 3.6 | 5.4 | 10.4 | 15.4 |
| iso-Pentane | 0.2 | 0.3 | 0.2 | 0.8 |

TABLE 4 (Continued)

| | Conventional HZSM—5 Extrudate with $SiO_2/Al_2O_3$ of 70/l | | | Highly Siliceous HZSM—5 Extrudate with $SiO_2/Al_2O_3$ of 1600/l |
|---|---|---|---|---|
| Example No. | 3 | 4 | 5 | 6 |
| Pentene | 3.4 | 5.1 | 10.1 | 14.3 |
| n-Pentane | <0.1 | <0.1 | 0.1 | 0.3 |
| $C_6^+$ | 32.1 | 43.5 | 55.5 | 65.9 |
| $C_5$ Olefin Distribution, wt.% | | | | |
| 1-Pentene | 4 | 4 | 3 | 2 |
| 2-Pentenes | 35 | 35 | 25 | 19 |
| Methyl Butenes | 61 | 61 | 72 | 79 |
| $C_6^+$ Composition, Wt.% | | | | |
| Paraffins, Total | 16 | 9 | 8 | 7 |
| iso | 10 | 6 | 7 | 6 |
| normal | 6 | 3 | 1 | 1 |
| Olefins | 77 | 88 | 89 | 93 |
| Naphthenes | 4 | 2 | 2 | <1 |
| Aromatics | 3 | 1 | 1 | <1 |
| Liquid Product 90% B.P. (D—2887) | 484°F | 434°F | 411°F | 388°F |
| Liquid Product Octane Number (R+O) | — | — | 91.3 | 94.3 |

**Claims**

1. An improved process for producing a gasoline fraction having no more than about 20 percent by weight of aromatics, said process comprising contacting a $C_2$—$C_5$ olefin containing mixture with a catalyst comprising a zeolite having a silica to alumina mole ratio of at least 200 to 1 and a constraint index in the approximate range of 1 to 12 at a temperature between 450°F (233°C) and 800°F (427°C), an olefin partial pressure between 0.5 psia (4 k Pa) and 100 psia 689 (k Pa) and a WHSV between 0.1 and 25, and recovering a gasoline fraction containing predominantly higher olefins.

2. A process according to Claim 1 wherein said zeolite is ZSM—5, ZSM—11, ZSM—12 or ZSM—48.

3. A process according to Claim 1 or Claim 2 wherein said silica to alumina mole ratio is at least 500 to 1.

4. A process according to any preceding Claim wherein said silica to alumina mole ratio is 1000 to 1.

5. A process according to any preceding Claim wherein said silica to alumina mole ratio is at least 1400 to 1.

6. A process according to any preceding Claim wherein said silica to alumina mole ratio is at least 1600 to 1.

7. A process according to any preceding Claim which is carried out at a temperature between 500°F (260°C) and 700°F (372°C), an olefin partial pressure between 1 psia (7 kPa) and 50 psia (345 kPa) and a WHSV between 0.5 and 15.

8. A process according to any preceding Claim wherein said zeolite is in the hydrogen form.

9. A process according to any preceding Claim wherein the gasoline fraction has a research octane number with no added lead of 92 or above.

10. A process according to any preceding Claim wherein said zeolite is incorporated in a matrix.

11. A process according to Claim 10 wherein the amount of zeolite in said matrix is from 1 wt.% to 95 wt.% of the total.

12. A process according to Claim 11 wherein the amount of zeolite is from 20 wt.% to 80 wt.% of the total.

13. A process according to any preceding Claim wherein said catalyst is in the extrudate form.

14. The process according to any preceding Claim wherein said olefin containing mixture comprises propylene and butylene.

**Revendications**

1. Procédé amélioré pour produire une fraction d'essence ne présentant pas plus qu'environ 20% en poids de produits aromatiques, ce procédé consistant à mettre en contact un mélange renfermant des oléfines en $C_2$ à $C_5$ avec un catalyseur comportant une zéolite présentant un rapport molaire de silice à alumine d'au moins 200 à 1 et un indice de contrainte dans la plage approximative de 1 à 12 à une température entre 233°C et 427°C, un pression partielle d'oléfine entre 4 kPa et 689 kPa et une VSHP entre 0,1 et 25, et à récupérer une fraction d'essence renfermant de façon prédominante des oléfines supérieures.

2. Un procédé selon la revendication 1, dans lequel ladite zéolite est ZSM—5, ZSM—11, ZSM—12 ou ZSM—48.

3. Un procédé selon la revendication 1 ou la revendication 2, dans lequel ledit rapport molaire de silice à alumine est d'au moins 500 à 1.

4. Un procédé selon l'une quelconque des revendications précédentes, dans lequel ledit rapport molaire de silice à alumine est de 1000 à 1.

5. Un procédé selon l'une quelconque des revendications précédentes, dans lequel ledit rapport molaire de silice à alumine est d'au moins 1400 à 1.

6. Un procédé selon l'une quelconque des revendications précédentes, dans lequel ledit rapport molaire de silice à alumine est d'au moins 1600 à 1.

7. Un procédé selon l'une quelconque des revendications précédentes, qui est mis en oeuvre à une température entre 260°C et 372°C, à une pression partielle d'oléfine entre 7 kPa et 345 kPa et une VSHP entre 0,5 et 15.

8. Un procédé selon l'une quelconque des revendications précédentes, dans lequel ladite zéolite est sous la forme hydrogène.

9. Un procédé selon l'une quelconque des revendications précédentes, dans lequel la fraction d'essence présente un indice d'octane de recherche sans addition de plomb de 92 ou au-dessus.

10. Un procédé selon l'une quelconque des revendications précédentes, dans lequel ladite zéolite est incorporée dans une matrice.

11. Un procédé selon la revendication 10, dans lequel la quantité de zéolite dans ladite matrice est de 1% en poids à 95% en poids du total.

12. Un procédé selon la revendication 11, dans lequel la quantité de zéolite est de 20% en poids à 80% en poids du total.

13. Un procédé selon l'une quelconque des revendications précédentes, dans lequel ledit catalyseur est sous la forme d'extrudat.

14. Le procédé selon l'une des revendications précédentes, dans lequel ledit mélange renfermant les oléfines comporte du propylène et du butylène.

**Patentansprüche**

1. Verbessertes Verfahren zur Erzeugung eine Benzinfraktion mit nicht mehr als etwa 20 Gew.-% Aromaten, dadurch gekennzeichnet, daß man ein $C_2$—$C_5$-Olefin-enthaltendes Gemisch mit einem Katalysator, der einen Zeolithen mit einem Siliciumdioxid/Aluminiumoxid-Molverhältnis von wenigstens 200 zu 1 und einem Zwangsindex etwa im Bereich von 1 bis 12 enthält, bei einer Temperatur zwischen 233°C (450°F) und 427°C (800°F), einem Olefin-Partialdruck zwischen 4 kPa (0,5 psia) und 689 kPa (100 psia) und bei einem WHSV-Wert zwischen 0,1 und 25 in Berührung bringt und eine Benzinfraktion, die vorweigend höhere Olefine enthält, gewinnt.

2. Verfahren nach Anspruch 1, dadurch gekennzeichnet, daß der Zeolith ZSM—5, ZSM—11, ZSM—12 oder ZSM—48 ist.

3. Verfahren nach Anspruch 1 oder 2, dadurch gekennzeichnet, daß das Siliciumdioxid/Aluminiumoxid-Molverhältnis wenigstens 500 zu 1 ist.

4. Verfarhen nach einem der vorhergehenden Ansprüche, dadurch gekennzeichnet, daß das Siliciumdioxid/Aluminiumoxid-Molverhältnis 1000 zu 1 ist.

5. Verfahren nach einem der vorhergehenden Ansprüche, dadurch gekennzeichnet, daß das Siliciumdioxid/Aluminiumoxid-Molverhältnis wenigstens 1 400 zu 1 ist.

6. Verfahren nach einem der vorhergehenden Ansprüche, dadurch gekennzeichnet, daß das Siliciumdioxid/Aluminiumoxid-Molverhältnis wenigstens 1 600 zu 1 ist.

7. Verfahren nach einem der vorhergehenden Ansprüche, dadurch gekennzeichnet, daß es bei einer Temperatur zwischen 260°C (500°F) und 372°C (700°F), einem Olefin-Partialdruck zwischen 7 kPa (1 psia) und 345 kPa (50 psia) und bei einem WHSV-Wert zwischen 0,5 und 15 ausgeführt wird.

8. Verfarhen nach einem der vorhergehenden Ansprüche, dadurch gekennzeichnet, daß der Zeolith in der Wasserstofform vorliegt.

9. Verfahren nach einem der vorhergehenden Ansprüche, dadurch gekennzeichnet, daß die Benzinfraktion eine Research-Octanzahl ohne Bleizugabe von 92 oder darüber hat.

10. Verfahren nach einem der vorhergehenden Ansprüche, dadurch gekennzeichnet, daß der Zeolith in ein Matrixmaterial einverleibt ist.

11. Verfahren nach Anspruch 10, dadurch gekennzeichnet, daß die Zeolithmenge in dem Matrixmaterial 1 Gew.-% bis 95 Gew.-% des Gesamten ist.

12. Verfahren nach Anspruch 11, dadurch gekennzeichnet, daß die Zeolithmenge 20 Gew.-% bis 80 Gew.-% des Gesamten ist.

13. Verfahren nach einem der vorhergehenden Ansprüche, dadurch gekennzeichnet, daß der Katalysator in Extrudatform vorliegt.

14. Verfahren nach einem der vorhergehenden Ansprüche, dadurch gekennzeichnet, daß das olefinhaltige Gemisch Propylen und Butylen enthält.